# EUROPÄISCHE PATENTSCHRIFT

(11) **EP 0 709 362 B1**
(45) Veröffentlichungstag und Bekanntmachung des Hinweises auf die Patenterteilung: **24.06.1998**
(21) Anmeldenummer: 95116240.3
(22) Anmeldetag: 16.10.1995
(51) Int. Cl.: C07C 67/303, C11C 3/12, C07C 69/30

(54) **Verfahren zur Hydrierung ungesättigter Fettsäureester**
Process for the hydrogenation of unsaturated fatty acid esters
Procédé d'hydrogénation d'esters d'acides gras insaturés

(30) Priorität: 28.10.1994 DE 4438547
(43) Veröffentlichungstag der Anmeldung: 01.05.1996
(73) Patentinhaber: BAYER AG, 51368 Leverkusen (DE)
(72) Erfinder: Darsow, Gerhard, Dr., D-47804 Krefeld (DE)

(56) Entgegenhaltungen:
- EP-A- 0 120 122

## Beschreibung

Die vorliegende Erfindung betrifft ein neues, kostengünstiges, kontinuierlich arbeitendes Verfahren zur Hydrierung von ungesättigten Fettsäureestern oder Fettsäureestergemischen zu gesättigten oder teilweise gesättigten Fettsäureestern oder Fettsäureestergemischen, bei welchem keine unerwünschten höheren Mono-Alkohole oder Aldehyde, die sich durch einen unangenehmen Geruch oder Geschmack auszeichnen, als Nebenprodukte gebildet werden.

Durch die Hydrierung ungesättigter Fettsäureester oder Fettsäureestergemische lassen sich aus den normalerweise flüssigen ungesättigten Verbindungen, feste mehr oder minder hoch schmelzende Verbindungen oder Verbindungsgemische herstellen.

Aus flüssigen pflanzlichen oder tierischen Gemischen von Fettsäureglyceriden lassen sich auf diese Weise hochwertige feste Nahrungsmittelfette gewinnen, die als Margarine oder Bratfette eine umfangreiche Verwendung finden, aber auch industriell (z.B. als Schmierstoffe) genutzt werden können.

Es ist bekannt, ungesättigte Fettsäureestergemische diskontinuierlich mit Wasserstoff über Ni-Pulver zu gesättigten Fettsäureestern zu hydrieren (DRP 141 029).

Es ist weiterhin bekannt, ungesättigte Fettsäureestergemische diskontinuierlich mit Wasserstoff über Mischkatalysatoren der Hydroxide, Oxide oder Carbonate von Ni, Co, Fe mit Cu oder Pd, Pt oder Ag zu gesättigten Fettsäureestern zu hydrieren (US 1 268 692).

Es ist ferner bekannt, ungesättigte Fettsäureestergemische teilkontinuierlich mit Wasserstoff in mehreren hintereinander geschalteten diskontinuierlichen Apparaten mit Ni-Pulvern oder Ni auf pulverförmiger Kieselgur als Träger zu hydrieren (GB 804 604, US 2 932 658).

Außerdem ist es bekannt, ungesättigte Fettsäureestergemische kontinuierlich mit Wasserstoff an stationär in einer senkrechten Kolonne befindlichen Ni-Spiralen zu hydrieren (GB 162 370, GB 203 218).

Der Reaktionsverlauf läßt sich z.B. für die Hydrierung von Linolsäuremethylester zu Stearinsäuremethylester durch das folgende Reaktionsschema veranschaulichen:

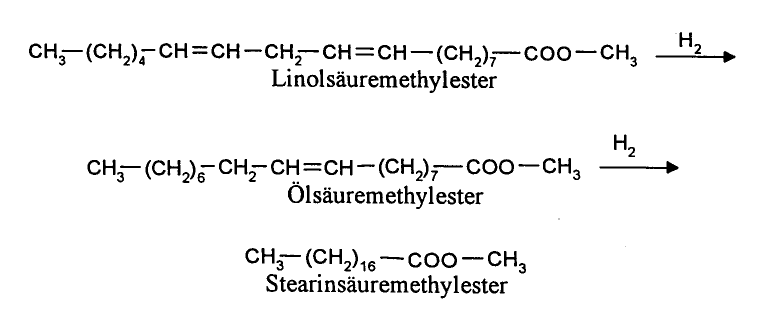

Bei den bekannten Verfahren zur Hydrierung ungesättigter Fettsäureester oder Fettsäureestergemische werden fast ausschließlich diskontinuierliche Suspensionsverfahren (Batch-Prozesse) angewandt, bei denen die Fettsäureester oder Fettsäureestergemische über pulverförmigen, überwiegend Ni-haltigen Katalysatoren mit Wasserstoff hydriert werden.

Diskontinuierliche Verfahren haben den Nachteil, daß ihre Kapazität relativ zum Reaktionsvolumen sehr klein ist und somit ein Bedarf nach großen Reaktionsapparaturen und Lagertanks besteht. Energieverbrauch und Personalbedarf sind verhältnismäßig hoch.

Kontinuierliche Pulverkatalysatorverfahren, die mit mehreren in Kaskade geschalteten Hydrierreaktoren arbeiten, vermeiden einen Teil dieser Nachteile. Es bleibt jedoch das Erfordernis, die pulverförmigen Katalysatoren mehrfach gezielt zu dosieren, umzupumpen und quantitativ vom Reaktionsprodukt abzufiltrieren. Die Katalysatorschlammpumpen unterliegen einem hohen mechanischen Verschleiß. Die quantitative Entfernung der pulverförmigen Katalysatoren aus dem Reaktionsprodukt ist aufwendig. Ferner ist die Gefahr groß, die Katalysatoraktivität durch die zusätzlichen Operationen verhältnismäßig schnell zu verringern. Es ist daher vorteilhaft, die Reaktion über fest angeordneten Katalysatoren ablaufen zu lassen. Solche Katalysatoren müssen eine hohe Aktivität besitzen, die über einen längeren Zeitraum nicht nachlassen darf, weil häufige Katalysatorwechsel bei Festbettreaktionen ebenfalls aufwendig sind.

Als kontinuierlich arbeitendes Verfahren wurde bisher die Hydrierung ungesättigter Fettsäureestergemische an stationär in einer senkrechten Kolonne befindlichen Ni-Spiralen (siehe oben) beschrieben. Ein solches Verfahren arbeitet jedoch wenig effektiv und hat sich auch in der Praxis nicht bewährt, weil die metallischen Ni-Spiralen nur eine verhältnismäßig kleine aktive Oberfläche (< 1 m³/g) aufweisen.

Überraschenderweise wurde nun gefunden, daß man ungesättigte Fettsäureester oder Fettsäureestergemische sehr gut kontinuierlich über im Festbett angeordneten trägerfreien Formkörpern aus sauerstofffreien Metallpulvern oder kleinen Metallteilchen, z.B. Granulaten, von einem oder mehreren Elementen der Eisenuntergruppe der VIII. Nebengruppe des Periodensystems (Mendelejew) zu gesättigten oder teilweise gesättigten Fettsäureestern oder Fettsäureestergemischen hydrieren kann. Dazu kann es nützlich sein, die Metalle der Eisenuntergruppe mit aktivierend wirkenden Elementen der VI. Nebengruppe des Periodensystems zu legieren. Dabei können die zum Einsatz kommenden Pulver oder Teilchen zusätzlich kleine Anteile nicht katalytisch wirkender Elemente (z.B. Silizium, Aluminium, Titan, Kohlenstoff) enthalten, ohne daß die hohe Aktivität gemindert wird. Die Festkörper müssen eine Druckfestigkeit von 20 - 250 N und eine innere Oberfläche von 10-90 m²/g aufweisen.

Zum Einsatz kommen reine ungesättigte Fettsäureester oder natürliche pflanzliche oder tierische Fettsäureestergemische.

Gegenstand der Erfindung ist damit ein Verfahren zur kontinuierlichen Herstellung von teilweise oder vollständig gesättigten Fettsäureestern oder Gemischen mehreren von ihnen durch katalytische Hydrierung ungesättigter Fettsäureester oder Gemischen mehrerer von ihnen, wobei der Säureteil der Ester 6 - 30 C-Atome enthält und der Alkoholteil ein- bis dreiwertig ist und 1 - 20 C-Atome enthält, das dadurch gekennzeichnet ist, daß die Hydrierung in der flüssigen Phase bei einem H₂-Druck von 50 - 350 bar, einer 20 - 60-fachen molaren H₂-Menge, bezogen auf die stöchiometrische Menge, und bei einer Temperatur von 40 - 150°C an im Festbett angeordneten sauerstofffreien und trägerfreien Katalysatoren durchgeführt wird, die als verpreßte aus Metallpulvern oder Metallteilchen hergestellte Formkörper vorliegen, die eine Druckfestigkeit von 20 - 250 N und eine innere Oberfläche von 10 - 90 m²/g aufweisen und bei denen die Metallpulver 65 - 100 Gew.-% eines oder mehrerer Eisenmetalle, 0 - 15 Gew.-% eines oder mehrerer Metalle der VI. Nebengruppe und 0 - 20 Gew.-% eines oder mehrerer hydrierinerter Elemente aus der Gruppe von Aluminium, Silicium, Titan und Kohlenstoff, alles bezogen auf das Gesamtgewicht des Metallpulvers, enthalten.

Die Druckfestigkeit der trägerfreien Formkörper kann nach DIN 50 106 bestimmt werden.

Die Überprüfung von trägerfreien Formkörpern auf die anspruchsgemäßen inneren Oberflächen und damit auf Brauchbarkeit für das erfindungsgemäße Verfahren können nach Methoden durchgeführt werden, die von F.M. Nelsen und F.T. Eggertsen, Analyt. Chem. 30 (1958), S. 1387-1390 bzw. S.J. Gregg und K.S.W. Sing, Adsorption, Surface Area and Porosity, London 1982, Kap. 2 und 6, beschrieben worden sind.

Die Eisenuntergruppe der VIII. Nebengruppe des Periodensystems (Mendelejew) enthält die Elemente Eisen, Kobalt und Nickel. Die erfindungsgemäß zu verwendenen trägerfreien Formkörper enthalten eines oder mehrere dieser Metalle in Mengen von mindestens 65, vorzugsweise mindestens 70, insbesondere mindestens 80 Gew.-%, bezogen auf das Gesamtgewicht der trägerfreien Formkörper.

Die VI. Nebengruppe des Periodensytems enthält die Elemente Chrom, Molybdän und Wolfram. Die erfindungsgemäß zu verwendenden trägerfreien Formkörper enthalten eines oder mehrerer dieser Metalle in Mengen von 0 - 15 Gew.-%. Für den Fall ihres Vorliegens enthalten die Metallpulver mindestens 0,1, vorzugsweise mindestens 0,3, insbesondere mindestens 0,5 Gew.-%, bezogen auf trägerfreie Formkörper; sie enthalten eines oder mehrere dieser Metalle in Mengen von höchstens 15, vorzugsweise höchstens 10 und insbesondere höchstens 5 Gew.-%, bezogen auf trägerfreie Formkörper.

Die erfindungsgemäß zu verwendenen trägerfreien Formkörper können darüber hinaus - jeweils bezogen auf trägerfreie Formkörper - bis zu 20, vorzugsweise bis zu 15 Gew.-% andere Elemente enthalten; Beispiele solcher Elemente, die nicht katalytisch wirken, umfassen Aluminium, Silicium, Titan und Kohlenstoff. Nach einer bevorzugten Ausführungsform enthalten die trägerfreien Formkörper außer den Metallen der VIII. und VI. Nebengruppe nicht mehr als 10 Gew.-% Aluminium und nicht mehr als 5 Gew.-% andere Elemente.

Für den Hydrierprozeß wird auf einen Druck von 50 - 350 bar, bevorzugt 100 bis 300 bar, vorkomprimierter reiner Wasserstoff eingesetzt, wobei man mit einer 20-bis 60-fachen, bevorzugt 20- bis 40-fachen molaren Wasserstoffmenge, bezogen auf die stöchiometrische Menge, arbeitet.

Die Hydrierung erfolgt kontinuierlich im Festbettverfahren an den als Hydrierungskatalysatoren dienenden trägerfreien Formkörpern der beschriebenen Art, indem man die zu hydrierenden ungesättigten Fettsäureester oder Fettsäureestergemische entweder im Gleichstrom mit dem zuvor zugemischten Wasserstoff von unten nach oben aufsteigend über die in den Hydrierreaktor gefüllten Formkörper strömen läßt oder auch dem von oben einströmenden Wasserstoff von unten kommend entgegenführt (Gegenstromverfahren).

Der Hydrierprozeß wird bei Temperaturen von 40 bis 150°C durchgeführt. Niedrigere Temperaturen bedingen höhere Verweilzeiten oder den Verzicht auf einen quantitativen Umsatz. Höhere Temperaturen führen zur Bildung unerwünschter Fettsäurealkohole.

Die stündliche Katalysatorbelastung kann 200 bis 1 000 g Fettsäureester oder Fettsäureestergemisch/l Katalysator betragen.

Der Hydrierreaktor kann entweder ein einzelnes Hochdruckrohr aus Stahl oder einer Stahllegierung sein, das mit den trägerfreien Formkörpern ganz oder teilweise gefüllt wird, wobei auch die Anwendung auf Horden (Drahtkörbe o.ä.) nützlich sein kann, oder aber ein ummanteltes Hochdruckrohrbündel, dessen Einzelrohre mit Formkörpern ganz oder teilweise gefüllt werden.

Die Herstellung der trägerfreien Formkörper kann nach gebräuchlichen Methoden durch Verpressen der Metallpulver oder Metallteilchen auf Tablettier- und Pelletiermaschinen unter hohem Druck erfolgen, wobei zur Verbesserung des Haftvermögens der Metallpartikel auch Graphit in Mengen von 0,5 - 1,5 Gew.-%, bezogen auf das Gesamtgewicht der den Katalysator bildenden Bestandteile, oder Klebestoffe in kleinen Mengen zum Einsatz kommen können. Die Herstellung der trägerfreien Formkörper erfolgt vorzugsweise in einer sauerstofffreien Atmosphäre, um Oberflächenoxidationen zu vermeiden. Am wirksamsten und für die Reaktionsführung am günstigsten sind tablettierte und pelletierte Formkörper mit Durchmessern von 3 bis 7 mm. Von erheblicher Bedeutung ist die Druckfestigkeit der Formkörper, die erfindungsgemäß bei Werten von 20 bis 250 N, bevorzugt 100 bis 220 N auf die gewölbte Formkörperoberfläche, liegt. Niedrigere Druckfestigkeiten führen zu Formkörperzerfall bzw. erosivem Abrieb, was eine metallische Kontaminierung des Reaktionsproduktes bewirken würde. Höhere Werte bedingen einen unangemessenen Aufwand beim Verpressen, ohne daß weitere Vorteile erzielt werden. Von erheblicher Bedeutung ist weiterhin die innere Oberfläche der Formkörper, die erfindungsgemäß bei Werten von 10 bis 90 m²/g liegt und ausschlaggebend für einen möglichst quantitativen Umsatz der Einsatzstoffe ist.

Unter den geschilderten Reaktionsbedingungen sind auf diese Weise ganz unerwartet hohe Katalysatorstandzeiten von 15 000 Stunden und mehr zu erzielen, was zu Katalysatorverbräuchen < 0,1 Gew.-%, bezogen auf hergestelltes Reaktionsprodukt, führt.

Das den Hydrierreaktor verlassende Reaktionsgemisch wird entspannt, wobei man den überschüssigen Wasserstoff abfangen und nach erfolgtem Komprimieren und Ergänzung von verbrauchtem Wasserstoff erneut zum Einsatz bringen kann. Bei einer kompletten Hydrierung besteht das Reaktionsgemisch zu mehr als 99 Gew.-% aus gesättigten Fettsäureestern oder Fettsäureestergemischen.

Ist nur eine teilweise Hydrierung der vorhandenen Doppelbindungen beabsichtigt, so lassen sich die teilhydrierten Fettsäureester oder Fettsäurestergemische in Abhängigkeit von der Reaktionstemperatur gemäß einem vorgebenen beabsichtigten Erstarrungspunkt gewinnen.

Die erfindungsgemäß einzusetzenden sauerstofffreien und trägerfreien Festbettkatalysatoren neigen im Gegensatz zu trägerhaltigen Katalysatoren nicht zum "Ausbluten" d.h. nicht zum Übergang von Katalysatorbestandteilen in ionischer oder kolloidaler Form in die Lösungsphase des Substrats, so daß das Substrat nicht durch Schwermetalle kontaminiert wird, die normalerweise ebenfalls nur mühsam, beispielsweise mit Hilfe von Ionenaustauschern, aus dem Substrat erntfernt werden können. Die einzusetzenden Katalysatormetalle können, etwa nach längerem Gebrauch des Katalysators leicht aufgearbeitet und wiederverwendet werden, da die Schwermetalle nicht umständlich von einem Trägermaterial getrennt werden müssen. Bei polyfunktionellen Verbindungen, beispielsweise bei nur teilweise veresterten mehrwertigen Alkoholen, war weiterhin die Neigung zu befürchten, daß mit Schwermetallionen komplexe Chelatverbindungen der Fettseifen gebildet werden, die nur schwierig aus den Estern oder Estergemischen entfernt werden können; diese Befürchtung tritt mit den erfindungsgemäß einzusetzenden Katalysatoren nicht ein.

Die erzeugten gänzlich oder teilweise hydrierten Fettsäureester oder Fettsäureestergemische weisen einen Gehalt an Katalysatorbestandteilen < 1 ppm auf und sind daher ohne jede weitere Reinigung im Lebensmittelbereich einsetzbar.

### Beispiele

### Beispiel 1

Ein senkrecht stehendes, wärmeisoliertes Hochdruckrohr aus nichtrostendem Stahl von 45 mm Innendurchmesser und 1 m Länge wurde mit 1,4 l eines durch Tablettierung von Ni-Pulver hergestellten Hydrierungskatalysators gefüllt, der bei einer Zylinderhöhe von 5 mm und einem Durchmesser von 5 mm eine Druckfestigkeit von 147 N auf die Zylindermantelfläche und eine innere Oberfläche von 33 m²/g aufwies. Durch dieses Rohr wurden stündlich 320 g reiner Linolsäuremethylester (>99 Gew.-%) gemeinsam mit der 20-fachen molaren Menge von unter einem Druck von 300 bar stehendem, hochreinem Wasserstoff von unten nach oben aufsteigend gepumpt.

Linolsäuremethylester und Wasserstoff wurden vorab gemeinsam durch einen Wärmeaustauscher geführt und so erhitzt, daß sie in das Hochdruckrohr mit einer Temperatur von 120°C eintraten. Das das Hochdruckrohr verlassende Gemisch aus flüssigem Reaktionsprodukt und überschüssigem Wasserstoff wurde in einen Abscheider geführt, von wo der Wasserstoff nach Ersatz der verbrauchten Menge wieder zusammen mit neuem Linolsäuremethylester in den Vorwärmer und von dort erneut in das Hochdruckrohr gepumpt wurde.

Die farblose, Klare und geruchlose Schmelze des Reaktionsproduktes wurde nach Abkühlung auf eine Temperatur <60°C und Entspannung auf Normaldruck gaschromatographisch untersucht. Sie enthielt keine ungesättigten Anteile mehr (Iodzahl: <0,1).

Der Gehalt an Stearinsäuremethylester lag bei > 99 Gew.-%, der Erstarrungspunkt bei 37/38°C.

Der Katalysator war nach einer Laufzeit von 4 800 Stunden unverändert wirksam, so daß sich die Zusammensetzung des Reaktionsproduktes über diesen Zeitraum nicht veränderte.

### Beispiel 2

In einem Hochdruckrohr wie in Beispiel 1 wurde bei einer Temperatur von 120°C und einem Wasserstoffdruck von 300 bar stündlich eine Menge von 280 g Sojaöl (Iodzahl: 121, Säurezahl: <0,1) hydriert. Der Katalysator wurde durch Tablettierung einer pulverisierten Ni/Al/Si-Legierung mit einem Al-Gehalt von 5,4 Gew.-% und einem Si-Gehalt von 0,2 Gew.-% gewonnen.

Die Tabletten hatten bei einer Zylinderhöhe von 3 mm und einem Durchmesser von 3 mm eine Druckfestigkeit von 148 N auf die Zylindermantelfläche und eine innere Oberfläche von 61 m²/g.

Nach einer Laufzeit von 3 300 Stunden lag der Umsatz des eingesetzten Sojaöls bei >99,0 Gew.-%. Das erhaltene Reaktionsprodukt war farblos und geruchlos und wies einen Erstarrungspunkt von 61°C sowie eine Iodzahl <1 und eine Säurezahl <0,1 auf. Der Ni/Al/Si-Gehalt lag bei <1 ppm.

### Beispiel 3

In einem Hochdruckrohr wie in Beispiel 1 wurde bei einer Temperatur von 55°C und einem Wasserstoffdruck von 300 bar stündlich eine Menge von 750 g Sojaöl (Iodzahl: 121, Säurezahl: <0,1) hydriert. Der Katalysator wurde durch Tablettierung einer pulverisierten Ni/Al-Legierung mit einem Al-Gehalt von 4,1 Gew.-% gewonnen.

Die Tabletten hatten bei einer Zylinderhöhe von 3 mm und einem Durchmesser von 3 mm eine Druckfestigkeit von 142 N auf die Zylindermantelfläche und eine innere Oberfläche von 68 m²/g. Das erhaltene Reaktionsprodukt war farblos und geruchslos und wies einen Erstarrungspunkt von 36°C sowie eine Iodzahl von 22 und eine Säurezahl <0,1 auf. Der Ni/Al-Gehalt im Reaktionsprodukt lag bei <0,1 ppm.

Der Katalysator war nach einer Laufzeit von 5 400 Stunden unverändert wirksam, so daß sich die Zusammensetzung des Reaktionsproduktes über diesen Zeitraum nicht veränderte.

### Beispiel 4

In einem Hochdruckrohr wie in Beispiel 1 wurde bei einer Temperatur von 85°C und einem Wasserstoffdruck von 200 bar der Wasserstoff im umgekehrten Reaktionsfluß wie in Beispiel 1 aufsteigendem Sonnenblumenöl (Iodzahl: 128, Säurezahl: <0,19) entgegengeführt, wobei stündlich eine gleichgroße Menge wie in Beispiel 1 hydriert wurde. Der Katalysator war durch Tablettierung einer pulverisierten Ni/Fe-Legierung gewonnen worden. Die Legierung enthielt einen Eisenanteil in Nickel von 15 Gew.-%. Die Tabletten hatten bei einer Zylinderhöhe von 5 mm und einem Durchmesser von 5 mm eine Druckfestigkeit von 137 N auf die Zylindermantelfläche und eine innere Oberfläche von 74 m²/g.

Die farblose, klare und geruchslose Schmelze des Reaktionsproduktes wurde nach Abkühlung auf eine Temperatur <60°C und Entspannung auf Normaldruck isoliert und wies einen Erstarrungspunkt von 56°C sowie eine Iodzahl von 2 und eine Säurezahl <0,1 auf. Der Ni/Fe-Gehalt der Schmelze betrug <0,1 ppm.

Der Katalysator war nach einer Laufzeit von 1 200 Stunden unverändert wirksam, so daß sich die Zusammensetzung des Reaktionsproduktes über diesen Zeitraum nicht veränderte.

### Beispiel 5

In einem Hochdruckrohr wie in Beispiel 1 wurde bei einer Temperatur von 48°C und einem Wasserstoffdruck von 300 bar stündlich eine Menge von 750 g Sonnenblumenöl (Iodzahl. 128, Säurezahl: 0,1) hydriert. Der Katalysator war durch Tablettierung einer pulverisierten Ni/Fe-Legierung gewonnen worden. Die Legierung enthielt einen Fe-Anteil in Ni von 15 Gew.-%. Die Tabletten hatten bei einer Zylinderhöhe von 5 mm und einem Durchmesser von 5 mm eine Druckfestigkeit von 137 N auf die Zylindermantelfläche und eine innere Oberfläche von 74 m²/g.

Die farblose, klare und geruchslose Schmelze des Reaktionsproduktes wurde nach Abkühlung auf eine Temperatur <40°C und Entspannung auf Normaldruck isoliert und wies einen Erstarrungspunkt von 32°C sowie eine Iodzahl von 26 und eine Säurezahl <0,1 auf. Der Ni/Fe-Gehalt der Schmelze betrug <0,1 ppm.

Der Katalysator war nach einer Laufzeit von 2 600 Stunden unverändert wirksam.

### Beispiel 6

Ein senkrecht stehendes, wärmeisoliertes Hochdruckrohr aus nichtrostendem Stahl von 45 mm Innendurchmesser und 1 m Länge wurde mit 1,4 l eines durch Tablettierung von Pulver einer Ni/Mo-Legierung mit einem Mo-Gehalt von 1,75 % hergestellten Hydrierungskatalysators gefüllt, der bei einer Zylinderhöhe von 5 mm und einem Durchmesser von 5 mm eine Druckfestgkeit von 191 N auf die Zylindermantelfläche und eine innere Oberfläche von 58 m²/g aufwies. Durch dieses Rohr wurden stündlich 400 g Rapsöl (Iodzahl: 102,5, Säurezahl: <1) gemeinsam mit der 30-fachen molaren Menge von unter einem Druck von 300 bar stehendem hochreinen Wasserstoff von unten nach oben aufsteigend gepumpt.

Rapsöl und Wasserstoff wurden vor Eintritt in das Hochdruckrohr auf eine Temperatur von 90°C gebracht.

Die farblose, klare und geruchslose Schmelze des Reaktionsproduktes wurde nach Abkühlung auf eine Temperatur <60°C und Entspannung auf Normaldruck isoliert und wies einen Erstarrungspunkt von 55°C sowie eine Iodzahl von 17,6 und eine Säurezahl <1 auf. Der Ni/Mo-Gehalt der Schmelze betrug <0,1 ppm.

Der Katalysator war nach einer Laufzeit von 2 800 Stunden unverändert wirksam, so daß sich die Zusammensetzung des Reaktionsproduktes über diesen Zeitraum nicht veränderte.

### Beispiel 7

In einem Hochdruckrohr wie in Beispiel 1 wurden bei einer Temperatur von 110°C und einem Wasserstoffdruck von 300 bar stündlich 360 g eines charakteristisch riechenden Rizinusöls (Iodzahl: 84, Säurezahl: 4, Hydroxylzahl: 6) hydriert. Der Katalysator wurde durch Tablettierung von Pulver einer Ni/Mo/Al-Legierung mit einem Mo-Gehalt von 1,02 Gew.-% und einem Al-Gehalt von 5,1 Gew.-% hergestellt. Die Tabletten hatten bei einer Zylinderhöhe von 5 mm und einem Durchmesser von 5 mm eine Druckfestigkeit von 210 N auf die Zylindermantelfläche und eine innere Oberfläche von 71 m²/g.

Das erhaltene Reaktionsprodukt war farblos und geruchlos und wies bei einem Erstarrungspunkt von 76°C eine Iodzahl von 7 und eine Hydroxylzahl von 6 auf. Der Ni/Mo-Gehalt lag bei <0,1 ppm.

Der Katalysator war nach einer Laufzeit von 2 400 Stunden unverändert wirksam.

### Beispiel 8

In einem Hochdruckrohr wie in Beispiel 1 wurde bei einer Temperatur von 55°C und einem Wasserstoffdruck von 300 bar Rizinusöl (Iodzahl: 84, Säurezahl: 4, Hydroxyzahl: 6) in einer gleich große Menge wie in Beispiel 1 hydriert. Der Katalysator wurde durch Tablettierung einer pulverisierten Ni/W-Legierung gewonnen. Die Legierung hatte einen W-Gehalt von 1,4 %. Die Tabletten hatten bei einer Zylinderhöhe von 3 mm und einem Durchmesser von 3 mm eine Druckfestigkeit von 162 N auf die Zylindermantelfläche und eine innere Oberfläche von 68 m²/g.

Das erhaltene Reaktionsprodukt war farblos und geruchlos und wies bei einem Erstarrungspunkt von 38°C eine Iodzahl von 18 und eine Säurezahl von 4 auf.

Der Katalysator war nach einer Laufzeit von 1 900 Stunden unverändert wirksam.

## Patentansprüche

1. Verfahren zur kontinuierlichen Herstellung von teilweise oder vollständig gesättigten Fettsäureestern oder Gemischen mehrerer von ihnen durch katalytische Hydrierung ungesättigter Fettsäureester oder Gemischen mehrerer von ihnen, wobei der Säureteil der Ester 6 bis 30 C-Atome enthält und der Alkoholteil ein- bis dreiwertig ist und 1 bis 20 C-Atome enthält, dadurch gekennzeichnet, daß die Hydrierung in der flüssigen Phase bei einem H₂-Druck von 50 bis 350 bar, einer 20 bis 60-fachen molaren H₂-Menge, bezogen auf die stöchiometrische Menge, und bei einer Temperatur von 40 bis 150°C an im Festbett angeordneten sauerstofffreien und trägerfreien Katalysatoren durchgeführt wird, die als verpreßte aus Metallpulvern oder Metallteilchen hergestellte Formkörper vorliegen, die eine Druckfestigkeit von 20 bis 250 N auf die gewölbte Formkörperoberfläche und eine innere Oberfläche von 10 bis 90 m²/g aufweisen und bei denen die Metallpulver 65 bis 100 Gew.-% eines oder mehrerer Eisenmetalle, der VIII. Nebengruppe des Periodensystems, 0 bis 15 Gew.-% eines oder mehrerer Metalle der VI. Nebengruppe und 0 bis 20 Gew.-% eines oder mehrerer hydrierinerter Elemente aus der Gruppe von Aluminium, Silicium, Titan und Kohlenstoff, alles bezogen auf das Gesamtgewicht des Metallpulvers, enthalten.

2. Verfahren nach Anspruch 1, dadurch gekennzeichnet, daß die Metallpulver oder Metallteilchen 65 bis 100 Gew.-%, bevorzugt 70 bis 100 Gew.-%, besonders bevorzugt 80-100 Gew.-% eines oder mehrerer Eisenmetalle enthalten.

3. Verfahren nach Anspruch 1, dadurch gekennzeichnet, daß die Metallpulver oder Metallteilchen beim Vorliegen von Metallen der VI. Nebengruppe einen Gehalt von 0,1 bis 15 Gew.-%, bevorzugt von 0,3 bis 10 Gew.-%, besonders bevorzugt von 0.5 bis 5 Gew.-%, daran enthalten.

4. Verfahren nach Anspruch 1, dadurch gekennzeichnet, daß die Metallpulver oder Metallteilchen beim Vorliegen von hydrierinerten Elementen einen Gehalt von 0 bis 10 Gew.-% an Aluminium und von 0 bis 5 Gew.-% je Element Si, Ti und C enthalten.

5. Verfahren nach Anspruch 4, dadurch gekennzeichnet, daß der Gesamtgehalt der hydrierinerten Elemente 0 bis 15 Gew.-%, bevorzugt 0 bis 10 Gew.-%, beträgt.

6. Verfahren nach Anspruch 1, dadurch gekennzeichnet, daß die Formkörper zylinder- oder kugelförmig sind und Durchmesser von 3 bis 7 mm besitzen.

7. Verfahren nach Anspruch 1, dadurch gekennzeichnet, daß es sich bei den Formkörpern um solche mit einer Druckfestigkeit von 100 bis 220 N auf die gewölbte Formkörperoberfläche handelt.

8. Verfahren nach Anspruch 1, dadurch gekennzeichnet, daß die Hydrierung bei einem H₂-Druck von 100 bis 300 bar durchgeführt wird.

9. Verfahren nach Anspruch 1, dadurch gekennzeichnet, daß bei einer 20 bis 40-fachen molaren H₂-Menge gearbeitet wird.

10. Verfahren nach Anspruch 1, dadurch gekennzeichnet, daß der zu hydriende ungesättigte Fettsäureester den Hydrierreaktor von unten nach oben aufsteigend passiert, während der für die Hydrierung benötigte Wasserstoff entweder gemeinsam mit dem ungesättigten Ester in den Reaktor gepumpt wird oder diesem, von oben nach unten strömend, entgegengeführt wird.

## Claims

1. Process for the continuous preparation of esters of partially or completely saturated fatty acids or mixtures of a plurality thereof by catalytic hydrogenation of esters of unsaturated fatty acids or mixtures of a plurality thereof, where the acid moiety of the esters contains 6 to 30 C atoms and the alcohol moiety is monohydric to trihydric and contains 1 to 20 C atoms, characterized in that the hydrogenation is carried out in the liquid phase at an H₂ pressure of 50 to 350 bar, a 20 to 60-times molar amount of H₂, based on the stoichiometric amount and at a temperature of 40 to 150°C on oxygen-free and support-free catalysts arranged in a fixed bed which are present as pressed shaped bodies produced from metal powders or metal particles which have a compressive strength of 20 to 250 N on the curved shaped body surface and an internal surface area of 10 to 90 m²/g and in which the metal powders contain 65 to 100% by weight of one or more ferrous metals of subgroup VIII of the Periodic Table, 0 to 15% by weight of one or more metals of subgroup VI and 0 to 20% by weight of one or more hydrogenation-inert elements selected from the group consisting of aluminium, silicon, titanium and carbon, all based on the total weight of the metal powder.

2. Process according to Claim 1, characterized in that the metal powders or metal particles contain 65 to 100% by weight, preferably 70 to 100% by weight, particularly preferably 80-100% by weight of one or more ferrous metals.

3. Process according to Claim 1, characterized in that the metal powders or metal particles, when metals of subgroup VI are present, contain these at 0.1 to 15% by weight, preferably 0.3 to 10% by weight, particularly preferably 0.5 to 5% by weight.

4. Process according to Claim 1, characterized in that the metal powders or metal particles, when hydrogenation-inert elements are present, have a content of 0 to 10% by weight of aluminium and 0 to 5% by weight of each of the elements Si, Ti and C.

5. Process according to Claim 4, characterized in that the total content of the hydrogenation-inert elements is 0 to 15% by weight, preferably 0 to 10% by weight.

6. Process according to Claim 1, characterized in that the shaped bodies are cylindrical or spherical and have diameters of 3 to 7 mm.

7. Process according to Claim 1, characterized in that the shaped bodies are those having a compressive strength of 100 to 220 N on the curved shaped body surface.

8. Process according to Claim 1, characterized in that the hydrogenation is carried out at an H₂ pressure of 100 to 300 bar.

9. Process according to Claim 1, characterized in that a 20 to 40-times molar H₂ amount is employed.

10. Process according to Claim 1, characterized in that the unsaturated fatty acid ester to be hydrogenated passes through the hydrogenation reactor ascending from bottom to top, while the hydrogen required for the hydrogenation is either pumped into the reactor together with the unsaturated ester or is conducted in the opposite direction to this flowing from top to bottom.

## Revendications

1. Procédé de préparation continue d'esters d'acides gras partiellement ou totalement saturés ou de mélanges de plusieurs de ceux-ci par hydrogénation catalytique d'esters d'acides gras insaturés ou de mélanges de plusieurs de ceux-ci, où la partie acide des esters contient 6 à 30 atomes de carbone et la partie alcool est mono- à trivalente et contient 1 à 20 atomes de carbone, caractérisé en ce que l'hydrogénation est réalisée en phase liquide à une pression de H₂ de 50-350 bars, avec une quantité de H₂ de 20-60 fois molaire, par rapport à la quantité stoechiométrique, et à une température de 40-150°C sur des catalyseurs exempts d'oxygène et exempts de support disposés en lit fixe, qui sont sous forme de corps façonnés comprimés formés à partir de poudres métalliques ou de particules métalliques, qui présentent une résistance à la pression de 20-250 N sur la surface bombée des corps façonnés et une surface intérieure de 10-90 m²/g et dans lesquels les poudres métalliques contiennent 65-100% en masse d'un ou plusieurs métaux de type fer du VIIIème groupe secondaire du système périodique, 0-15% en masse d'un ou plusieurs métaux du VIème groupe secondaire et 0-20% en masse d'un ou plusieurs éléments inertes vis-à-vis de l'hydrogénation du groupe de l'aluminium, du silicium, du titane et du carbone, le tout étant rapporté à la masse totale de la poudre métallique.

2. Procédé selon la revendication 1, caractérisé en ce que les poudres métalliques ou les particules métalliques contiennent 65 à 100% en masse, de préférence 70 à 100% en masse, de manière particulièrement préférée 80 à 100% en masse d'un ou plusieurs métaux de type fer.

3. Procédé selon la revendication 1, caractérisé en ce que, dans le cas où des métaux du VIème groupe secondaire sont présents, les poudres métalliques ou les particules métalliques en contiennent une teneur de 0,1 à 15% en masse, de préférence de 0,3 à 10% en masse, de manière particulièrement préférée de 0,5 à 5% en masse.

4. Procédé selon la revendication 1, caractérisé en ce que, dans le cas où des éléments inertes à l'égard de l'hydrogénation sont présents, les poudres métalliques ou les particules métalliques contiennent une teneur de 0 à 10% en masse d'aluminium et de 0 à 5% en masse de chacun des éléments Si, Ti et C.

5. Procédé selon la revendication 4, caractérisé en ce que la teneur totale des éléments inertes à l'égard de l'hydrogénation est de 0 à 15% en masse, de préférence de 0 à 10% en masse.

6. Procédé selon la revendication 1, caractérisé en ce que les corps façonnés sont de forme cylindrique ou sphérique et possèdent des diamètres de 3 à 7 mm.

7. Procédé selon la revendication 1, caractérisé en ce que, concernant les corps façonnés, il s'agit de corps façonnés ayant une résistance à la pression de 100 à 220 N sur la surface bombée des corps façonnés.

8. Procédé selon la revendication 1, caractérisé en ce que l'hydrogénation est réalisée à une pression de H₂ de 100 à 300 bars.

9. Procédé selon la revendication 1, caractérisé en ce que l'on opère avec une quantité de H₂ 20 à 40 fois molaire.

10. Procédé selon la revendication 1, caractérisé en ce que l'ester d'acide gras insaturé à hydrogéner traverse le réacteur d'hydrogénation en montant de bas en haut tandis que l'hydrogène nécessaire pour l'hydrogénation est pompé dans le réacteur en même temps que l'ester insaturé ou est conduit à contre-courant de celui-ci en circulant de haut en bas.
